(19) Europäisches Patentamt
European Patent Office
Office européen des brevets

(11) Veröffentlichungsnummer: **0 000 373**
**B1**

(12) **EUROPÄISCHE PATENTSCHRIFT**

(21) Anmeldenummer: **78100314.0**

(22) Anmeldetag: **06.07.78**

(51) Int. Cl.³: **C 07 D 233/90**, C 07 D 403/02, C 07 D 413/02, A 01 N 43/50

(54) **Imidazolcarbonsäuren und deren Derivate, Verfahren zu ihrer Herstellung und ihre Verwendung zur Pflanzenwachstumsregulierung bzw. zum Pflanzenschutz**

(30) Priorität: **19.07.77 DE 2732531**

(43) Veröffentlichungstag der Anmeldung: **24.01.79 Patentblatt 79/02**

(45) Bekanntmachung des Hinweises auf die Patenterteilung: **07.01.81 Patentblatt 81/01**

(84) Benannte Vertragsstaaten: **BE CH DE FR GB NL SE**

(56) Entgegenhaltungen: **DE - A - 1 545 988**

(73) Patentinhaber: **HOECHST AKTIENGESELLSCHAFT**
**Postfach 80 03 20**
**D - 6230 Frankfurt/Main 80 (DE)**

(72) Erfinder: **Söder, Alfons, Dr.**
**Kauber Weg 1**
**D - 6000 Frankfurt/Main (DE)**
**Bieringer, Hermann, Dr.**
**Eichenweg 26**
**D - 6239 Eppstein**
**Taunus (DE)**
**Bürstell, Helmut, Dr.**
**Am Dachsberg 89**
**D - 6000 Frankfurt/Main (DE)**
**Langelüddeke, Peter, Dr.**
**Nelkenweg 5**
**D - 6238 Hofheim am Taunus (DE)**
**Sachse, Burkhard, Dr.**
**Parkstrasse 18**
**D - 6233 Kelkheim am Taunus (DE)**

D - 6000 Frankfurt ain (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 373 B1

Imidazolcarbonsäuren und deren Derivate, Verfahren zu ihrer Herstellung und ihre Verwendung zur Pflanzenwachstumsregulierung bzw. zum Pflanzenschutz

Aus der DE-OS 2 130 673 sind Benzhydryl-imidazolderivate mit Alkylsubstitution im Imidazolring bekannt. Ebenso wurden bereits 1-Benzyl- und 1-Tetrahydronaphthyl-imidazol-(5)-carbonsäuren und deren Ester beschrieben (J. Med. Chem. *15*, 336—337 (1972)). Benzhydryl-imidazolderivate mit einer Carboxylfunktion im Imidazolrest sind bisher nicht bekannt.

Es wurde gefunden, daß derartige Verbindungen wertvolle Eigenschaften als Pflanzenschutzmittel und Pflanzenwachstrumsregulatoren besitzen.

Gegenstand der Erfindung sind demnach Verbindungen der Formeln

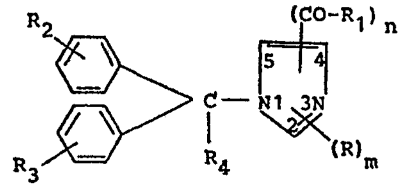

(I)

bzw.

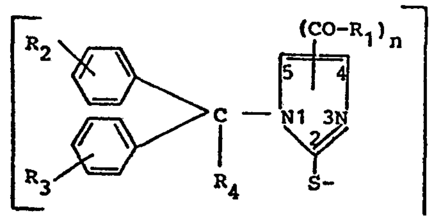

(II)

worin

$m = 0 - 2$; $n = 1$ oder 2 und $m + n \leq 3$;

R Halogen, $(C_1—C_6)$Alkyl, Allyl, Hydroxy$(C_1—C_6)$alkyl, Halogen$(C_1—C_6)$alkyl, Mercapto, $(C_1—C_6)$Alkylthio, Cyano, Phenyl oder Phenyl$(C_1—C_2)$alkyl;

$R_1$ Hydroxy, $(C_1—C_6)$Alkoxy, Hydroxy$(C_2—C_6)$alkoxy, $(C_2—C_6)$-Alkoxy-alkoxy, Di$(C_1—C_3)$alkylphosphinyl-$(C_1—C_3)$alkoxy, Di$(C_1—C_3)$alkylphosphinyl-$(C_2—C_3)$hydroxyalkoxy, $(C_1—C_3)$-Alkylthio, Amino, -$(C_1—C_6)$Alkylamino, Di$(C_1—C_6)$alkylamino, Di$(C_1—C_6)$alkylamino-$(C_1—C_3)$alkylamino, Hydroxyamino, $(C_1—C_3)$-Alkoxyamino; N-$(C_1—C_3)$-Alkyl-N-$(C_1—C_3)$-alkoxyamino, Anilino, N-Pyrrolidino, N-Piperidino, N-Morpholino, Hydrazino, N'-$(C_1—C_3)$-Alkylhydrazino, N',N'-Dimethylhydrazino oder N'-Phenylhydrazino,

$R_2$ und $R_3$ gleich oder verschieden sind und Wasserstoff, Halogen, $(C_1—C_3)$Alkyl, Trifluormethyl, Hydroxy, $(C_1—C_3)$Alkoxy, Halogen$(C_1—C_3)$alkoxy, $(C_1—C_3)$Alkylthio, Cyano, Nitro oder Acetamino, und

$R_4$ Wasserstoff oder Phenyl bedeuten sowie ihre nicht-toxischen Salze mit Säuren oder Basen.

In den Verbindungen der Formeln I und II steht "Halogen" vorzugsweise für Chlor oder Brom. Bei den Salzen der Verbindungen handelt es sich vorzugsweise um Säuresalze mit Mineralsäuren wie zum Beispiel Salpetersäure, Salzsäure, Schwefelsäure oder Phosphorsäure oder um Alkalimetall-(Na,K)- oder Ammoniumsalze; doch kommen auch Erdalkalisalze oder Salze mit organischen Basen wie Triäthylamin in Betracht.

Die erfindungsgemäßen Verbindungen können nach einer Vielzahl literaturbekannter Verfahren hergestellt werden. Diese Verfahren sind zum Beispiel dadurch gekennzeichnet, daß man

a) Verbindungen der Formel

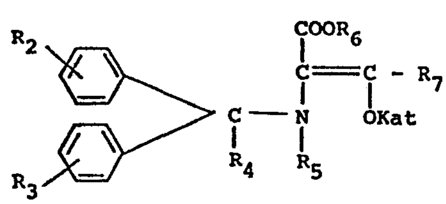

(III)

worin

$R_5$ Formyl oder Acetyl

$R_6$ $(C_1—C_6)$-Alkyl

$R_7$ Wasserstoff oder $(C_1—C_6)$Alkoxycarbonyl und Kat ein Alkalimetallkation bedeuten, mit Rhodanwasserstoffsäure umsetzt und gewünschtenfalls in den erhaltenen 2-Mercapto imidazolen der Formel

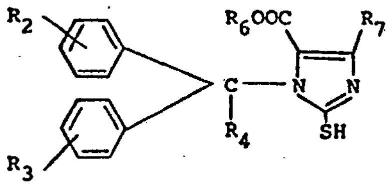

(IV)

die SH-Gruppe in 2-Stellung des Imidazolrings

$a_1$) unter Ausbildung einer -S-S-(Disulfid)-Brücke dehydriert oder

$a_2$) durch Entschwefeln eliminiert und anschließend gewünschtenfalls halogeniert oder in 2-Stellung hydroxymethyliert oder

b) Verbindungen der Formel

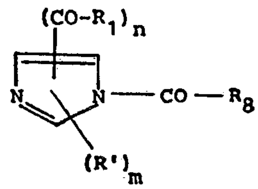

(V)

worin $R_8$ Wasserstoff oder ($C_1$—$C_3$)Alkyl bedeutet mit Verbindungen der Formel und R′ die Bedeutung von R mit Ausnahme der -SH-Gruppe besitzt

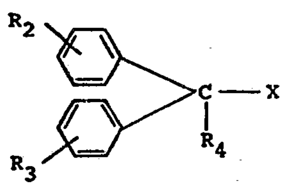

(VI)

worin X ein Halogenatom oder eine Alkyl- oder Arylsulfonylgruppe darstellt, umsetzt, anschließend die Gruppe -CO-$R_8$ in bekannter Weise abspaltet, oder

c) Verbindungen der Formel

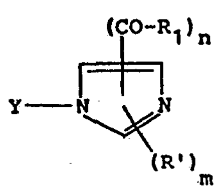

(VII)

worin Y Wasserstoff, ein Alkalimetall- oder Silberatom bedeutet, mit Verbindungen der Formel VI umsetzt, oder

d) Verbindungen der Formel

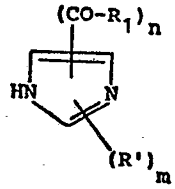

(VIII)

mit Verbindungen der Formel

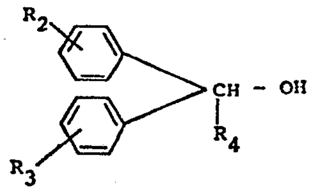

(IX)

umsetzt, oder

e) Verbindungen der Formeln

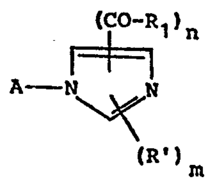

(X)

worin A eine Trimethylsilyl- oder Halogenmagnesiumgruppe bedeutet, mit Verbindungen der Formel

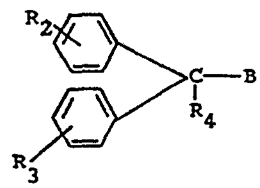

(XI)

worin B ein Halogenatom oder die Hydroxylgruppe darstellt, umsetzt oder

f) Verbindungen der Formel

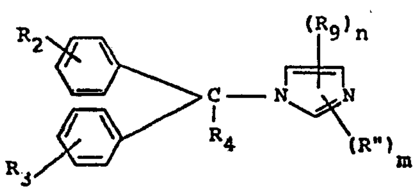

(XII)

worin R″ die Bedeutung von R mit Ausnahme der -SH- und der $(C_1$—$C_6)$Alkylthiogruppe besitzt und $R_9$ die Gruppen $-CH_2OH$ oder -CHO bedeutet, oxydiert, oder

g) in Verbindungen der Formel

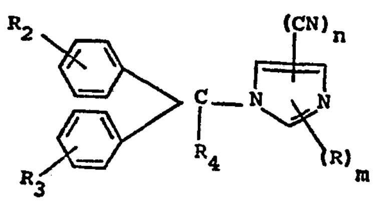

(XIII)

die -CN-Gruppe(n) zu(r) Carboxylgruppe(n) oder zu(r) Säureamidgruppe(n) hydrolysiert, und gewünschtenfalls in den nach a)—g) erhaltenen Verbindungen die Estergruppe umestert oder versieft und/oder die erhaltenen freien Säuren gewünschtenfalls in nicht phytotoxische Salze, Ester, Thiolester, Amide, Anilide oder Hydrazide der Formeln I bzw. II überführt bzw. (bei Vorhandensein von mehreren Estergruppen) eine davon thermisch abspaltet.

Die Herstellungsverfahren seien im folgenden näher beschrieben:

a) Die Ausgangsstoffe der Formel III sind durch Umsetzung von Benzhydrylamin mit Chloressigsäureester zum N-Benzhydrylglycinester, Formylierung bzw. Acetylierung des letzteren am Stickstoffatom und weitere Umsetzung der erhaltenen N-Acylverbindung mit Ameisensäureester ($R_7$ = H)

oder Oxalsäureester zugänglich (J. Am. Chem. Soc. *71*, 644 (1949)). Der Ringschluß mit Rhodanwasserstoffsäure erfolgt glatt in einem inerten wäßrigen System, z.B. Wasser oder dem Gemisch Wasser/Tetrahydrofuran, bei Temperaturen zwischen etwa 50° und dem Siedepunkt des Lösungsmittels.

Auf diese Weise entstehen Verbindungen der Formel I, die in 4-Stellung des Imidazolrings Wasserstoff oder eine Carbalkoxy gruppe und in 2-Stellung eine -SH-Gruppe enthalten. Letztere kann in bekannter Weise, beispiels weise mittels Jod, Sauerstoff, Wasserstoffperoxid oder Sulfurylchlorid dehydriert werden, wobie Disulfide der Formel III erhalten werden.

Aus den in erster Stufe erhaltenen Verbindungen kann ferner die SH-Gruppe durch Entschwefelung eliminiert werden, beispielsweise durch 15 prozentige Salpetersäure bei 30 bis 35°C oder Nickel bei 50—100°C.

Die entschwefelten Verbindungen können anschließend in 2-Stellung in üblicher Weise halogeniert oder hydroxymethyliert werden. Die Einführung der Hydroxymethylgruppe gelingt beispielsweise durch Anwendung von methanolischer oder wäßriger Formaldehydlösung bei erhöhter Temperatur, vorzugsweise 130 bis 140°C im Druckgefäß.

b) Bei dieser Reaktion dient die Gruppe -CO-$R_8$ als Schutzgruppe, die eine doppelte Reaktion an beiden Stickstoffatomen des Imidazolrings verhindert. Die Ausgangsstoffe der Formel V sind durch Acylierung von Verbindungen der Formel VIII am Stickstoffatom 1 erhältlich. Aus der Umsetzung der Verbindungen der Formel V mit denen der Formel VI resultiert eine Quaternierung am Stickstoffatom 3. Die nachfolgende Abspaltung der Acylgruppe durch Hydrolyse, Alkoholyse oder Aminolyse, die sehr leicht erfolgt, führt in bekannter Weise (E.F. Godefroi, J. H. F. M. Mentjens, Rec. Trav. Chim. Pays-Bas *93* (1974), 56) zu den gewünschten Verbindungen der Formel I.

In Formel VI steht X allgemein für eine die Quaternisierung des Imidazolrings ermöglichende Gruppe; als solche kommt vorzugsweise Halogen (Cl, Br) oder eine Alkyl- oder Arylsulfonylgruppe (Mesyl, Tosyl) in Betracht.

Die Umsetzung von V mit VI verläuft allgemein bei Temperaturen von 60° bis 100°C in einem geeigneten Lösungsmittel wie Acetonitril oder Propionitril.

c) Diese Reaktion entspricht dem Verfahren b) mit dem Unterschied, daß das zweite Stickstoffatom des Imidazolrings nicht geschützt ist. Um eine zweifache Reaktion (insbesondere wenn Y = H) zu vermeiden, ist es zweckmäßig, die Umsetzung in Gegenwart eines tertiären Amins (z.B. Triäthylamin oder N-Äthyl-diisopropyl-amin in einemLösungsmittel wie z.B. Acetonitril, Dimethylformamid, Dimethylsulfoxid, Chloroform, Tetrachlorkohlenstoff bei Temperaturen von 20° bis 85° durchzuführen.

d) Die Abspaltung von Wasser gemäß d) geschieht vorteilhaft bei Temperaturen von 130 bis 150°C in einem inerten Lösungsmittel wie Xylol oder Mesitylen, gegebenenfalls in Gegenwart von katalytischen Mengen von Aluminiumoxid.

e) Dagegen verläuft die Umsetzung nach e) bereits bei niedrigen Temperaturen von 0°C bis Raumtemperatur oder wenig darüber. Als Lösungsmittel verwendet man zweckmäßig wasserfreies Benzol, Toluol, Tetrahydrofuran oder Dioxan.

f) Die Ausgangsstoffe der Formel XII können gemäß J. Am. Chem. Soc. *71* (1949), 2801—2803 aus entsprechend substituierten Vorprodukten hergestellt werden. Die Oxidierung erfolgt in bekannter Weise mit einem üblichen Oxidationsmittel beispielsweise Kaliumpermanganat, Wasserstoffperoxid, Chromtrioxid, t-Butylchromat, Nickelperoxid, Silberoxid oder Mangandioxid bei Temperaturen von 0° bis 35°C. Als Lösungsmittel haben sich Ketone wie Aceton oder Methyläthylketon, Essigsäure, Dimethylsulfoxid oder Wasser bewährt.

g) Die Wasseranlagerung an Nitrilgruppen führt je nach den Verfahrensbedingungen entweder zu Carbonamiden oder Carbonsäuren. Um erstere zu erhalten, läßt man die Komponenten bei 20—50°C in 1n Natronlauge gegebenenfalls in Gegenwart katalytischer Mengen Wasserstoffperoxid reagieren; letztere entstehen bei der Verseifung der Nitrilgruppe(n) mit 6n Natronlauge bei 70° bis 100°C. Die Ausgangsstoffe der Formel XIII erhält man z.B. nach dem Verfahren von Bull. Chem. Soc. Japan *41* (1968), 1237—1240 aus entsprechend substituierten Vorprodukten.

Die gemäß den Varianten a) bis g) des erfindungsgemäßen Verfahrens erhaltenen Verbindungen können anschließend in mannigfacher Weise an der bzw. den -CO-$R_1$-Gruppe(n) abgewandelt werden. Beispielsweise können Ester in bekannter Weise zu freien Säuren bzw. deren Salzen verseift werden. Freie Säuren, wie sie nach a) bis f) durch Verseifung von Estergruppen oder nach g) durch Verseifung von Cyangruppen entstehen, können weiterhin, gegebenenfalls auf dem Weg über die Säurechloride, in andere Ester, Thiolester, Amide, Anilide oder Hydrazide der Formel -CO-$R_1$ überführt werden. Auch kann bei Vorhandensein von zwei Estergruppen eine davon verseift und decarboxyliert werden. All diese Verfahren sind dem Fachmann bekannt und bedürfen keiner näheren Erläuterung.

## Beispiel 1

a) *1-Benzhydryl-2-mercapto-5-methoxycarbonyl-imidazol*

Unter Rühren wurden in ein Gemisch von 200 ml Tetrahydrofuran, 200 ml Wasser und 16,5 ml einer 36-prozentigen wäßrigen Salzsäure 31,3 g (0,1 Mol) 2-(Benzhydryl-formyl)-amino-3-oxo-propionsäure-methylester (hergestellt durch Formylierung von (Benzhydryl-formyl)-amino-essigsäuremethylester nach R. G. Jones, J. Am. Chem. Soc. *71* (1949), 644) und 18 g Kaliumthioxyanat

eingetragen. Nach 3 1/2-stündigem Rühren bei 40°C wurde die organische Phase abgetrennt und das Lösungsmittel im Vakuum entfernt. Der Rückstand wurde in Methylenchlorid aufgenommen und durch zweimaliges Ausschütteln mit Wasser von Säureresten befreit. Nach dem Waschen mit Methanol hinterblieben 29,8 g (92% der Theorie) 1-Benzhydryl-2-mercapto-5- methoxycarbonyl-imidazol, Schmelzpunkt 194°C.

Analog Beispiel 1 a) wurde erhalten:

1 b) 1-[Phenyl - 4 - chlorphenyl) - methyl] - 2 - mercapto - 5 - methoxycarbonyl - imidazol, Schmelzpunkt 184°C.

1 c) 1-[Phenyl-4-methylphenyl)-methyl)]-2-mercapto-5-methoxycarbonyl-imidazol, Fp. 186°.

Beispiel 2

a) *1-Benzhydryl-2-mercapto-5-imidazol-carbonsäure*

3,2 g (0,01 Mol) 1-Benzhydryl-2-mercapto-5-methoxycarbonyl-imidazol (Beispiel 1 a) wurden mit 20 ml einer 1n Natronlauge drei Stunden auf 80°C erhitzt, wobei in praltisch quantitativer Ausbeute 1-Benzhydryl-2-mercapto-5-imidazol-carbonsäure-Di-natriumsalz erhalten wurde (Ausbeute 3,5 g). Das Salz zersetzt sich oberhalb 255°C. Durch Lösen des Salzes in Wasser und Ansäurern der Lösung mit 2n Salzsäure erhält man die kristalline 1-Benzhydryl-2-mercapto-5-imidazol-carbonsäure vom Schmelzpunkt 158°C.

Analog Beispiel 2 a) wurde erhalten:

b) 1-[(Phenyl-4-chlorphenyl)-methyl]-2-mercapto-5-imidazol-Schmelzpunkt 153°C.

c) 1-[(Phenyl-4-methylphenyl)-methyl]-2-mercapto-5-imidazolcarbonsäure, Fp 140°.

Beispiel 3

a) *1-Benzhydryl-2-mercapto-5-propoxycarbonyl-imidazol*

3,1 g (0,01 Mol) 1-Benzhydryl-2-mercapto-5-imidazolcarbonsäure (Beispiel 2 a)) wurden mit 100 ml 1-Propanol unter Einleiten von Salzsäuregas bis zur Beendigung der Veresterung auf 97°C erwärmt. Bei der basischen Aufarbeitung wurden 3,3 g (94% der Theorie) 1-Benzhydryl-2-mercapto-5-propoxycarbonyl-imidazol vom Schmelzpunkt 133°C erhalten.

Analog Beispiel 3 a) wurde erhalten:

3 b) 1-Benzhydryl-2-mercapto-5-äthoxycarbonyl-imidazol, Schmelzpunkt 194°C.

Beispiel 4

*1-Benzhydryl-5-methoxycarbonyl-2-methylthio-imidazol*

3,2 g (0,01 Mol 1-Benzhydryl-2-mercapto-5-methoxycarbonyl-imidazol (Beispiel 1 a)) wurden in ein Gemisch von 30 ml Wasser, 15 ml Methanol, 15 ml Methylenchlorid, 10 ml 2n Natronlauge und 1,3 ml Methyljodid eingetragen. 30 Minuten wurde nachgerührt, wobei die Temperatur das Reaktionsgemisches auf 39°C anstieg. Nach dem Abkühlen wurden aus der Methylenchlorid-Phase 3 g (90% der Theorie) 1-Benzhydryl-5-methoxycarbonyl-2-methylthio-imidazol, mit einem Schmelzpunkt von 118°C isoliert.

Beispiel 5

*1-Benzhydryl-2-mercapto-5-imidazol-carbonsäure-pyrrolidid*

3,2 g (0,01 Mol) 1-Benzhydryl-2-mercapto-5-methoxycarbonyl-imidazol (Beispiel 1 a)) wurden mit 60 ml Pyrrolidin unter Stickstoff 3 Tage zum Sieden erhitzt. Darauf wurde der Überschuß an Pyrrolidin im Vakuum entfernt und das gebildete 1-Benzhydryl-2-mercapto-5-imidazol-carbonsäure-pyrrolidid aus Methanol umkristallisiert. Ausbeute: 3,3 g (91% der Theorie), Schmelzpunkt 263°C.

Beispiel 6

*Bis-(1-benzhydryl-5-methoxycarbonyl-imidazolyl(2))-disulfid*

Eine Lösung von 3,2 g (0,01 Mol) 1-Benzhydryl-2-mercapto-5-methoxycarbonyl-imidazol (Beispiel 1 a)) in 60 ml 2n Natronlauge wurde mit einer 10-prozentigen wäßrigen Kaliumjodid-Jod-Lösung bis zur Beendigung der Niederschlagsbildung versetzt. Der Niederschlag wurde isoliert, mit Wasser ausgewaschen und in einen Scheidetrichter mit 250 ml Tetrachlorkohlenstoff und 50 ml einer 5-prozentigen wäßrigen Natriumhydrogensulfit-Lösung überführt. Dabei gingen das gebildete Disulfid und etwas komplexgebundenes Jod in Lösung. Beim Einengen der organischen Phase kristalliesert das Bis-(1-benzhydryl-5-methoxycarbonyl-imidazolyl(2))-disulfid aus. Ausbeute: 3,0 g (95% der Theorie), Schmelzpunkt 154°C.

Beispiel 7

*Bis-(1-benzhydryl-5-carboxy-imidazolyl(2))-disulfid*

3.2 g (0,005 Mol) Bis-(1-benzhydryl-5-methoxy-carbonylimidazolyl(2))-disulfid (Beispiel 6) wurden mit der äquivalenten Menge 2n Natronlauge bei 80°C verseift. Beim Ansäurern des erkalteten Reaktionsgemischs mit Salzsäure wurden 3,0 (96% der Theorie) Bis-(1-benzhydryl-5-carboxy-imidazolyl(2))-disulfid vom Schmelzpunkt 180°C (Zers.) erhalten.

Beispiel 8

a) *1-Benzhydryl-5-methoxycarbonyl-imidazol*

Zu 325 ml Methylenchlorid wurden unter Rühren 325 ml einer 15-prozentigen Salpetersäure und 1,6 g Natriumnitrit zugegeben. Bei 35°C wurden unter weiteren Rühren portionsweise 32,5 g (0,1 Mol) 1-Benzhydryl-2-mercapto-5-methoxycarbonyl-imidazol (Beispiel 1 a)) getragen. Nach 30-minütigem Nachrühren bei 35°C wurde das Reaktionsgemisch auf 10°C abgekühlt und das auskristallisierte 1-Benzhydryl-5-methoxycarbonyl-imidazol-Nitrat abgesaugt. Das mit Eiswasser von Säureestern befreite und bei 30°C im Vakuum getrocknette Kristallisat zeigt einen Schmelzpunkt von 175°C (Zers.).

Aus der gewaschenen und neutralisierten Methylenchloridphase wurden beim Eindampfen 9,2 g (32% der Theorie) 1-Benzhydryl-5-methoxy-carbonyl-imidazol vom Schmelzpunkt 129°C gewonnen.

Zur Überfuhrung in die freie Base wurde das Nitrat in 100 ml Chloroform suspendiert. Nach Zugabe von ca. 60 ml einer 1n Natronlauge ging das gesamte Nitrat in Lösung. Aus der Chloroformlösung konnten 17,8 g (61% der Theorie) 1-Benzhydryl-5-methoxycarbonyl-imidazol isoliert werden.

In analoger Weise erhält man aus 1-[(Phenyl-4-chlorphenyl)-methyl]-2-mercapto-5-methoxycarbonyl-imidazol (Beispiel 1 b)).

b) 1-[(Phenyl-4-chlorphenyl)-methyl]-5-methoxycarbonyl-imidazol, Schmelzpunkt 94°C.

c) 1-[(Phenyl-4-methylphenyl)-methyl]-5-methoxycarbonyl-imidazol, Fp 85°.

Beispiel 9

*1-Benzhydryl-5-methoxycarbonyl-imidazol-hydrochlorid*

1 g 1-Benzhydryl-5-methoxycarbonyl-imidazol (Beispiel 8) wurde in 45 ml Diäthyläther gelöst. Durch Zugabe von ätherischer Salzsäure erhält man das 1-Benzhydryl-5-methoxycarbonyl-imidazol-hydrochlorid vom Schmelzpunkt 136°C.

Beispiel 10

*1-Benzhydryl-2-hydroxymethyl-5-methoxycarbonyl-imidazol*

2,9 g (0,01 Mol) 1-Benzhydryl-5-methoxycarbonyl-imidazol (Beispiel 8) wurden in 25 ml einer 50-prozentigen methanolischen Formaldehydlösung 36 Stunden auf 135°C erhitzt. Aus dem Reaktionsgemisch wurden in fast quantitativer Ausbeute 3,15 g 1-Benzhydryl-2-hydroxymethyl-5-methoxycarbonyl-imidazol vom Schmelzpunkt 160°C gewonnen.

Beispiel 11

a) *1-Benzhydryl-5-imidazol-carbonsäure*

2,9 g (0,01 Mol) 1-Benzhydryl-5-methoxycarbonyl-imidazol (Beispiel 8) wurden bei 80°C mit einer äquivalenten Menge einer 1n Natronlauge verseift. Das erhaltene 1-Benzhydryl-5-imidazol-carbonsäure-Natriumsalz schmilzt bei 176°C.

Beim Ansäuern einer wäßrigen Lösung des Natriumsalzes mit Salzsäure erhält man die gewünschte 1-Benzhydryl-5-imidazol-carbonsäure vom Schmelzpunkt 216°C Ausbeute: 2,64 g (95% der Theorie).

In analoger Weise erhält man aus 1-[(Phenyl-4-chlorphenyl)-methyl]-5-methoxycarbonyl-imidazol (Beispiel 6 b).

b) 1-[(Phenyl-4-chlorphenyl)-methyl]-5-imidazol-carbonsäure, Schmelzpunkt 135°C.

c) 1-[(Phenyl-4-methylphenyl)-methyl]-5-imidazol-carbonsäure, Fp. 202°C.

Aus dem getrockneten Na-Salz der 1-Benzhydryl-5-imidazol-carbonsäure (11 a)) erhält man

d) durch Umsetzung mit n-Butylbromid das 1-Benzhydryl-5-n-butoxy-carbonyl-imidazol. Fp. 53°,

e) durch Umsetzung mit Benzylchlorid das 1-Benzhydryl-5-benzyloxycarbonyl-imidazol, Fp. 104°.

Beispiel 12

a) *1-Benzhydryl-5-propoxycarbonyl-imidazol*

2,8 g (0,01 Mol) 1-Benzhydryl-5-imidazol-carbonsäure (Beispiel 11) wurden mit 140 ml 1-Propanol unter Einleiten von Salzsäuregas bis zur vollständigen Veresterung zum Sieden erhitzt. Die Isolierung und Freisetzung aus dem Hydrochlorid ergab 3,1 g (96% der Theorie) 1-Benzhydryl-5-propoxycarbonyl-imidazol vom Schmelzpunkt 81°C.

Analog Beispiel 12 a) wurde erhalten:

b) 1-Benzhydryl-5-äthoxycarbonyl-imidazol, Schmelzpunkt 92°C,

c) 1-Benzhydryl-5-n-butoxycarbonyl-imidazol, Fp. 53°,

d) 1-Benzhydryl-5-benzyloxycarbonyl-imidazol, Fp. 104°.

Beispiel 13

1-Benzhydryl-5-propoxycarbonyl-imidazol durch Umesterung 2,9 g (0,01 Mol) 1-Benzhydryl-5-methoxycarbonyl-imidazol (Beispiel 8) wurden in Gegenwart katalytischer Mengen Toluolsulfosäure mit 100 ml 1-Propanol einige Zeit zum Sieden erhitzt. Die erhaltenen farblosen Kristalle zeigten ebenfalls einen Schmelzpunkt von 81°C.

Beispiel 14

a) *1-Benzhydryl-5-(dimethyl-phosphinyl-methoxycarbonyl)-imidazol*

3,0 g (0,01 Mol) 1-Benzhydryl-5-imidazol-carbonsäure-Natriumsalz (Beispiel 11) wurden mit 1,8 g Chlormethyl-dimethyl-phosphinoxyd in 40 ml Dimethylformamid unter Stickstoff so lange auf 120°C erhitzt, bis die Umsetzung vollendet war. Danach wurde das Lösungsmittel im Vakuum entfernt. Durch Aufnehmen des Rückstandes in Aceton wurde unlösliches Natriumchlorid abgetrennt. Die Kristallisation ergab farblose Kristalle vom Schmelzpunkt 174°C.

Ausbeute: 3,2 g (88% der Theorie).

Analog Beispiel 14 a) wurde erhalten:

b) 1-Benzhydryl-5-(3-(dimethyl-phosphinyl)-propoxycarbonyl)-imidazol. Schmelzpunkt 78°C, unter Verwendung von 3-Chlorpropyl-dimethyl-phosphinoxid,

c) 1-Benzhydryl-5-(2-dimethylphosphinyl-2-hydroxy-äthoxycarbonyl)-imidazol, Schmelzpunkt 197°C, unter Verwendung von 1-Dimethyl-phosphinyl-2-chlor-äthanol und

d) 1-Benzhydryl-5-(2-dimethylphosphinyl)-2-hydroxy-propoxycarbonyl)-imidazol, Schmelzpunkt 89°C, unter Verwendung von 2-Dimethyl-phosphinyl-1-chlor-propanol(2).

Beispiel 15

a) *1-Benzhydryl-5-hydrazinocarbonyl-imidazol*

2,9 g (0,01 Mol) 1-Benzhydryl-5-methoxycarbonyl-imidazol (Beispiel 8) wurden in 10 ml Methanol mit 0,8 Hydrazinhydrat einige Zeit sum Sieden erhitzt. Nach dem Abkühlen wurden 2,9 g 1-Benzhydryl-5-hydrazincarbonyl-imidazol vom Schmelzpunkt 152°C isoliert.

Analog Beispiel 15 a) wurde erhalten:

b) 1-Benzhydryl-5-(N',N'-dimethyl-hydrazinocarbonyl)-imidazol vom Schmelzpunkt 211°C.

c) 1-Benzhydryl-5-(N'-methyl-hydrazinocarbonyl)-imidazol vom Schmelzpunkt 123°C und

d) 1-Benzhydryl-5-hydrazinocarbonyl-2-mercapto-imidazol vom Schmelzpunkt 222°C.

Beispiel 16

a) *1-Benzhydryl-5-imidazol-carbonsäure-pyrrolidid*

2,9 g (0,01 Mol) 1-Benzhydryl-5-methoxycarbonyl-imidazol (Beispiel 8) wurden in 15 ml Pyrrolidin 6 Stunden zum Sieden erhitzt. Dann wurde das überschüssige Pyrrolidin im Vakuum entfernt und der Rückstand in Methylenchlorid gelöst. Mit Hilfe einer Kieselgelsäule wurde das gebildete 1-Benzhydryl-5-imidazol-carbonsäure-pyrrolidid von gefärbten Verunreinigungen abgetrennt.

Ausbeute: 2,8 g (86% der Theorie), Schmelzpunkt 117°C.

Analog wurde erhalten:

b) 1-Benzhydryl-5-(2'-diäthylamino-äthylaminocarbonyl)-imidazol-monohydrat, Fp. 60°.

Beispiel 17

a) *1-Benzhydryl-5-imidazol-carbonsäure-amid*

2,9 g (0,01 Mol) 1-Benzhydryl-5-methoxycarbonyl-imidazol wurden in einem Druckgefäß 8 Stunden mit 300 ml einer bei 20° gesättigten methanolischen Ammoniaklössung auf 120°C erhitzt. Man erhielt 2,7 g (96% der Theorie) 1-Benzhydryl-5-imidazol-carbonsäure-amid vom Schmelzpunkt 153°C.

Analog Beispiel 17 a) wurde erhalten:

b) 1-Benzhydryl-2-mercapto-5-imidazol-carbonsäure-amid vom Schmelzpunkt 131°C (Reaktionszeit 24 Stunden).

Beispiel 18

*1-Benzhydryl-2-mercapto-4,5-di-methoxycarbonyl-imidazol*

36,9 g (0,01 Mol) 2-(Benzhydryl-formyl)-amino-3-oxo-bernsteinsäure-dimethylester (hergestellt durch Umsetzung von (Benzhydryl-formyl)-amino-essigsäuremethylester mit Oxalsäure-dimethylester in Gegenwart von Naturiummethylat wurden analog Beispiel 1 a) mit 18 g Kaliumthiocyanat umgesetzt. Hierbei beträgt die Reaktionszeit 48 Stunden bei 50°C. Das gebildete 1-Benzhydryl-2-mercapto-4,5-di-methoxycarbonyl-imidazol wurde durch Zugabe von Hexan zur Kristallisation gebracht.

Ausbeute: 33,2 g (87% der Theorie), Schmelzpunkt 154°C.

Beispiel 19

*1-Benzhydryl-4,5-di-methoxycarbonyl-imidazol*

3,8 g (0,01 Mol) 1-Benzhydryl-2-mercapto-4,5-di-methoxycarbonyl-imidazol (Beispiel 17) wurden in 50 ml Methanol in Gegenwart von 400 mg Ranex-Nickel 4 Stunden am Rückfluß erhitzt. Dann wurde der Katalysator abgetrennt und gut mit Methanol gewaschen. Beim Einengen der vereinigten Filtratt erhält man 3,1 g (90% der Theorie) 1-Benzhydryl-4,5-di-methoxycarbonyl-imidazol vom Schmelzpunkt 93°C.

Beispiel 20

a) *1-Benzhydryl-4,5-di-methoxycarbonyl-imidazol*

Zu einer Suspension von 18,4 g (0,01 Mol) 4,5-Dimethylcarbonyl-imidazol in 60 ml Acetonitril und 14 ml Triäthylamin wurden 20,3 g Benzhydrylchlorid tropfenweise zugegeben. Dann wurde das Reaktionsgemisch 8 Stunden auf 80°C erhitzt, danach das Lösungsmittel im Vakuum entfernt und das gebildete Triäthylammonium-chlorid mit Hilfe von Wasser abgetrennt. Aus Methanol wurden 30 g (85% der Theorie) 1-Benzhydryl-4,5-di-methoxycarbonyl-imidazol vom Schmelzpunkt 93°C erhalten.

Analog erhält man unter Verwendung von 2-Chlor-tritylchlorid (= 2-Chlortriphenylmethylchlorid).

b) 1-(2'Chlor-trityl)-4,5-di-methoxycarbonyl-imidazol, Schmelzpunkt 202°C.

Beispiel 21

*1-Benzhydryl-2-imidazol-carbonsäure-Kaliumsalz*

2,64 g (0,01 Mol) 1-Benzhydryl-2-hydroxymethyl-imidazol vom Schmelzpunkt 174°C (hergestellt aus 1-Benzhydryl-imidazol und Formaldehyd) werden in 150 ml Aceton gelöst. Dann wurde bei 5°C eine Lösung von 1,7 g Kaliumpermanganat in 120 ml Aceton unter Rühren tropfenweise zugegeben. Nach 2 Stunden wurde das Lösungsmittel im Vakuum entfernt, der Rückstand mit 150 ml Chloroform und 150 ml Wasser gerührt und von abgeschiedenem Braunstein abfiltriert. Aus der Chlorororm-Phase wurde kristallines 1-Benzhydryl-2-imidazol-carbonsäure-Kaliumsalz erhalten, das sich erst oberhalb 280°C zersetzt.

*1-Benzhydryl-2-imidazol-carbonsäure*

Das nach Beispiel 20 erhaltene Kaliumsalz lieferte beim Rühren mit 0,1 n Salzsäure die in feien Nadeln kristallisierende 1-Benzhydryl-2-imidazol-carbonsäure vom Schmelzpunkt 191°C in einer Ausbeute von ca. 70% der Theorie.

Beispiel 22

*1-Benzhydryl-5-methoxycarbonyl-imidazol*

1,7 g (0,01 Mol) 1-Acetyl-4-methoxycarbonyl-imidazol wurden in 5 ml Acetonitril mit 2 g Benzhydrylchlorid 4 Stunden zum Sieden erhitzt. Dann wurde das Lösungsmittel im Vakuum entfernt und der Rückstand mit 15 ml einer 0.5 n Natriumacetatlösung zur Abspaltung der Acetylgruppe gerührt. Aus Chloroform erhielt man das 1-Benzhydryl-5-methoxycarbonyl-imidazol vom Schmelzpunkt 129°C.

Beispiel 23

a) *1-Benzhydryl-4,5-imidazol-dicarbonsäure*

2,9 g (0,01 Mol) 1-Benzhydryl-4,5-imidazol-dinitril wurden mit einer äquivalenten Menge einer 3 n Natronlauge durchgreifend verseift. Beim Ansäuern der Lösung wurden 2,7 g (85% der Theorie) 1-Benzhydryl-4,5-imidazol-dicarbonsäure abgeschieden.

Schmelzpunkt 194°C.

Analog erhält man durch Versiefen von 1-(2'-Chlor-trityl)-4,5-imidazol-dinitril

b) 1-(2'-Chlor-trityl)-4,5-imidazol-dicarbonsäure, Schmelzpunkt 270°C (Zers.).

Beispiel 24

*1-Benzhydryl-5-methylthiocarbonyl-imidazol*

2,8 g (0,01 Mol) 1-Benzhydryl-5-imidazol-carbonsäure werden mit 4,5 ml Thionylchlorid bei 40°C in das Hydrochlorid des 1-Benzhydryl-5-imidazolcarbonsäure-chlorids überführt, das mit Hilfe von Methylenchlorid von restlichem Thionylchlorid befreit wird. Das kristalline Hydrochlorid wird nun in 20 ml Tetrahydrofuran unter Rühren mit 1,4 g (0,02 Mol) Natriummethylmercaptid bei 25°C über Nacht umgesetzt. Das Lösungsmittel wird im Vakuum entfernt und durch 50 ml Methylenchlorid ersetzt. Von unlöslichem Natriumchlorid wird die Lösung des Thiomethylesters abgetrennt. Der als öliger Rückstand aus der Lösung erhaltene Ester kristallisiert beim Durcharbeiten mit Pentan. Das in 85-prozentiger Ausbeute erhaltene 1-Benzhydryl-5-methylthiocarbonyl-imidazol zeigt einen Schmelzpunkt von 98°C.

Analog Beispiel 24 a) wurde aus dem 1-Benzhydryl-5-imidazolcarbonsäurechlorid-hydrochlorid folgende Verbindungen erhalten:

b) Durch Umsetzung mit Natrium-2-methoxyäthylat und anschließendes Ansäurern mit HCl das 1-Benzhydryl-5-(2-methoxyäthoxy)-carbonyl-imidazol-hydrochlorid, Fp. 125°,

c) durch Umsetzung mit Hydroxylamin-hydrochlorid + Triäthylamin 1-Benzhydryl-5-hydroxyaminocarbonyl-imidazol, Fp. 93°,

d) durch Umsetzung mit Diäthylamino-äthylamino-hydrochlorid Triäthylamin 1-Benzhydryl-5-(2-diäthylamino-äthylamino)-carbonyl-imidazol-monohydrat, Fp. 60°,

e) durch Umsetzung mit Na-propylmercaptid und Ansäuern mit HCl das 1-Benzhydryl-5-n-propylthiocarbonyl-imidazolhydrochlorid, Fp. 140°.

Beispiel 25

*1-Benzhydryl-2(4)-brom-5-methoxycarbonyl-imidazol*

2,9 g (0,01 Mol) 1-Benzhydryl-5-methoxycarbonyl-imidazol und 1,4 g feingepulvertes Kaliumcarbonat werden unter Rühren in 60 ml Tetrachlorkohlenstoff mit einer Lösung von 1,6 g Brom in 5 ml Tetrachlorkohlenstoff versetzt. 6 Sunden hält man das Reaktionsgemisch bei Siedetemperatur, läßt erkalten und dekantiert die Lösung von ausgeschiedenem Kaliumbromid ab. Nun ersetzt man das Lösungsmittel durch 15 ml Diisopropyläther, um das 1-Benzhydryl-2(4)-brom-5-methoxycarbonyl-imidazol in schwach-gelben Kristallen isolieren zu können. Man erhält 3,0 g (82% der Theorie) vom Schmelzpunkt 78°C.

Die erfindungsgemäßen Verbindungen sind als Pflanzenbehandlungsmittel in der Landwirtschaft und im Gartenbau vielseitig verwendbar. Sie sind wirksame Wachstumsregulatoren, Herbizide und Fungizide. Weiterhin können sie zur Bekämpfung von Moos, von pflanzenpathogenen Bakterien sowie als Antimykotika eingesetzt werden

Die Erfindung betrifft daher auch Mittel zum Einsatz in Landwirtschaft und Gartenbau sowie als Antimykotika, pflanzenwachstumsregulierende und herbizide Mittel sowie Mittel zur Bekämpfung von Pilzen und pflanzenpathogenen Bakterien.

Zur Herstellung der Mittel überführt man die erfindungsgemäßen Wirkstoffe in an sich bekannter Weise allein oder in Kombination mit weiteren Wirk- oder Nährstoffen in die üblichen Formulierungen, wie Pulver, Stäube, Pasten, Granulate, Lösungen, Schäume, Emulsionen und Suspensionen. Zur Vermischung und Streckung der Wirkstoffe eignen sich zum Beispiel Lösungsmittel, verflüssigte Gase, Emulgiermittel, Dispergiermittel, Schaumerzeuger und feste Trägermaterialien wie sie der Verarbeitung non Pflanzenschutzmitteln bzw. Pharmaka zur Verfügung stehen.

Die Verbindungen können innerhalb eines beträchtlichen Konzentrationsbereichs von etwa 0,00005 bis 2% angewendet werden. In besonderen Fällen können die Wirkstoffe auch in höherer Konzentration, ja sogar in reiner Form, z.B. mikrofein gemahlen, Verwendung finden. Bei der Anwendung als Herbizide oder Wachstumsregulatoren beträgt die Wirkstoffkonzentration pro ha Bodenfläche im allgemeinen 0,01 kg Wirkstoff. Bei der Formulierung der Wirkstoffe werden 20- bis 50-prozentige Spritzpulver, die die gebräuchlichen Anteile von Inertmitteln, Zellpech und Netzmitteln, ggf. auch Haftmittel enthalten, 15- bis 30-prozentige Emulsionskonzentrate sowie 5-prozentige Granulate neben Stäubemitteln verschiedener Wirkstoffkonzentrationen bevorzugt. Zubereitungen zur Behandlung von Hautpilzen enthalten in der Regel 0,5 bis 2% des Wirkstoffs.

Als Wachstumsregulatoren zeigen die erfindungsgemäßen Verbindungen eine ausgezeichnete wachstumshemmende Wirksamkeit z.B. bei Getreide, Ackerbohnen und Zierrasen sowie im Keimtest bei Leinsamen und Haferkörnern.

Mit Hilfe von Wachstumsregulatoren kann die Ernte erleichtert und der Ernteertrag gesteigert und gleichzeitig die Qualität der Ernteerzeugnisse gesteigert werden. Durch Halmverkürzung und -verstärkung bei Getriede wird die Nährstoffversorgung der Ähren verbessert und können Lagerverluste vermieden werden. Weiter läßt sich der Proteingehalt in Getreide und Soja sowie der Zuckergehalt in Zuckerrüben und -rohr durch Anwendung von Wachstumsregulatoren erhöhen. Andere Einsatzgebiete sind zum Beispiel die Optimierung der Stecklingsvermehrung und des Blattwachstums bei Tabakpflanzen. In Pflegebereichen kann das Wachstum von Rasen, Kräutern und Gehölzen gesteuert werden, wodurch sich die Phlegekosten senken lassen. In besonderen Fällen wird der Einsatz mechanischer Erntehilfen durch die Anwendung von Wachstumsregulatoren erst ermöglicht oder zumindest verbilligt. Im Zierpflanzenanbau läßt sich eine gute Anpassung an die qualitativen und zeitlichen Erfordernisse des Marktes erreichen.

Die erfindungsgemäßen Verbindungen besitzen außerdem—besonders im Vorauflauf—sehr gute herbizide Eigenschaften gegen eine große Reihe wirtschaftlich wichtiger Schadgräser und dikotyler Unkräuter. Sie sind andererseits verträglich gegenüber einigen landwirtschaftlich und gärtnerisch wichtigen Kulturen, wie Baumwolle, Mais, Raps, Bohnen usw., so daß sie sich zur selektiven Unkrautbekämpfung eignen.

Die Verbindungen haben ferner eine ausgezeichnete, teilweise systemische Wirkung gegen phytopathogene Pilze und eignen sich daher hervorragend als Pflanzenschutzmittel. Sie zeigen eine gute fungizide Wirkung z.B. gegen Rostpilze, Phytophthora infestans, Plasmopara viticola, Venturia inaequalis, Phoma betae und Botrytis cinerea sowie gegen Hautpilze wie Trichophton mentagrophytes und Microsporium canis.

Eine ausgezeichnete fungizide Wirkung weisen die Verbindungen gegen Piricularia oryzae und echte Mehltauarten an Gurke, Getreide (Weizen und Gerste), Äpfel und Zierpflanzen auf. Besonders hervorzuheben ist die hervorragende fungizide Wirkung der Verbindungen gegen Benzimidazol-restistente Mehltauarten.

Die fungiziden Mittel können in üblicher Weise z.B. als Stäube, Spritzpulver, Dispersionen und Emulsionskonzentrate formuliert werden. Ihr Gehalt an Gesamtwirkstoff beträgt vorzugsweise 10 bis 90 Gew-%. Daneben enthalten sie die üblichen Haft-, Netz-, Dispergier-, Füll- und Trägerstoffe.

Beispiel I (Wuchshemmung)

Junge Getreidepflanzen wurden im 3-Blatt-Stadium mit der Wirkstoffzubereitung tropfnaß

besprüht. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Die Ergebnisse sind in Tabelle 1 zusammengestellt. Es bedeuten 100% den Stillstand des Wachstums und 0% ein Wachstum entsprechend dem der unbehandelten Kontrollpflanzen.

TABELLE 1

Wuchshemmung bei Getreide

| Verbindung (Beispiel) | Konzentration (kg /ha) | Wuchshemmung in % Weizen | Gerste | Roggen |
|---|---|---|---|---|
| 6 | 2,5 | 25 | 28 | 40 |
| | 1,25 | 20 | 15 | 31 |
| 8 a | 2,5 | 41 | 36 | 42 |
| | 1,25 | 32 | 31 | 37 |
| Vergleich (Chloräthyl)-trimethylammonium-chlorid) | 2,5 | 31 | 10 | 10 |
| | 1,25 | 28 | 0 | 0 |

Beispiel II (herbizide Wirkung)

Unkräuter aus vielen botanischen Familien wurden in Töpfen ausgesät und dann mit den erfindungsgemäßen Verbindungen im Vorauflaufverfahren behandelt.

Ca. 4 Wochen nach Behandlung wurden die Unkräuter, die im Gewächshaus aufgelaufen und herangewachsen waren, visuell bonitiert nach dem Schema von Bolle (Tabelle 2). Hierbei zeigte sich, daß die untersuchten Verbindungen zahlreiche Unkräuter sehr gut bekämpfen.

Die Ergebnisse der visuellen Versuchsbonitur sind in folgenden Tabellen 3 und 4 dargestellt.

TABELLE 2

Boniturschema nach Bolle (Nachrichtenblatt des Deutschen Pflanzenschutzdienstes *16*, 1964, 92 – 94

| Wertzahl | Schadwirkung in % an Unkräutern | Kulturpflanzen |
|---|---|---|
| 1 | 100 | 0 |
| 2 | 97,5 bis 100 | 0 bis 2,5 |
| 3 | 95 ,, 97,5 | 2,5 ,, 5 |
| 4 | 90 ,, 95 | 5 ,, 10 |
| 5 | 85 ,, 90 | 10 ,, 15 |
| 6 | 75 ,, 85 | 15 ,, 25 |
| 7 | 65 ,, 75 | 25 ,, 35 |
| 8 | 32,5 ,, 65 | 35 ,, 67,5 |
| 9 | 0 ,, 32,5 | 67,5 ,, 100 |

TABELLE 3

Biologische Wirksamkeit gegen wichtige monokotyle und dikotyle Unkräuter im Vorauflaufverfahren

| Verbindung (Beispiel) | Dosis (kg /AS /ha) | Pflanzenart 1 | 2 | 3 | 4 | 5 | 6 | 7 | 8 | 9 | 10 | 11 |
|---|---|---|---|---|---|---|---|---|---|---|---|---|
| | 2.5 | - | 3 | 1 | 1 | 1 | 1 | 1 | 1 | 3 | 1 | 1 |
| 1 a | 0.6 | - | 4 | 1 | 1 | 2 | 1 | 1 | 1 | 4 | 1 | 1 |
| | 2.5 | 4 | 2 | 1 | 1 | 1 | 2 | 1 | 1 | 3 | 1 | 1 |
| 8 a | 0.6 | 4 | 2 | 1 | 1 | 1 | 2 | 1 | 1 | 3 | 1 | 1 |
| | 2.5 | - | 2 | 1 | 1 | 2 | 2 | 1 | 1 | - | 1 | 1 |
| 6 | 0.6 | - | 3 | 1 | 1 | 3 | 5 | 1 | 1 | - | 4 | 1 |

Legende:

1 Avena
2 Alopecurus
3 Setaria
4 Poa
5 Cyperus
6 Lolium
7 Digitaria
8 Echinochloa
9 Chrysanthemum
10 Stellaria
11 Amaranthus
AS = Aktivsubstanz

Beispiel III (fungizide Wirkung)

In den folgenden Beispielen stehen die Buchstaben A bis E für die nachstehend genannten Vergleichsmittel:

A: Methyl-1-(Butylcarbamoyl)-2-benzimidazol-carbamat (Benomyl)
B: N-tridecyl-2,6-dimethyl-morpholin (Tridemorph)
C: 2,4-Dinitro-6-sek.phenyl-3,3-dimethyl-acrylat (Binapacryl)
D: O-Äthyl-S,S-diphenyl-dithio-phosphat (Edifenfos)
E: Polyoxin

Weizenpflanzen wurden im 3-Blattstadium mit Koniden des Weizenmehltaus (Erysiphe graminis) stark inokuliert und in einem Gewächshaus bei 20°C und einer relativen Luftfeuchte von 90 bis 95% aufgestellt. In gleicher Weise wurden Gurkenpflanzen im 2-Blatzstadium und Reispflanzen im 4-Blattstadium mit Konidien von Erysiphe cichoracearum (Gurkenmehltau) bzw. Piricularia oryzae behandelt. 3 Tage nach Inokulation wurden die Pflanzen mit erfindungsgemäßen Verbindungen in verschiedenen Wirkstoffkonzentrationen tropfnaß gespritzt. Als Vergleichsmittel dienten A und B,

TABELLE 4

Wirksamkeit gegen Schadpilze

| Verbindung (Beispiel) | Konzentration (mg Wirkstoff pro 1 Spritzbrühe) | befallene Blattfläche in % bei Anwendung gegen | | |
|---|---|---|---|---|
| | | Erysiphe graminis (Weizen) | Erysiphe cichoracearum (Gurke) | Piricularis oryzae (Reis) |
| 1 a | 250 | 0 | 0 | – |
| | 125 | 0 | 0 | – |
| 2 a (Na-Salz) | 250 | – | 0 | – |
| | 125 | – | 0 | – |
| 6 | 250 | 0 – 3 | 0 | – |
| | 125 | 3 | 0 | – |
| 8 a | 250 | 0 – 3 | 0 | 0 – 3 |
| | 125 | 3 | 0 | 3 |
| A | 250 | 5 | 5 | – |
| | 125 | 10 | 10 | – |
| B | 250 | 5 | – | – |
| | 125 | 10 | – | – |
| C | 250 | – | 5 | – |
| | 125 | – | 15 | – |
| D | 250 | – | – | Phytotox. |
| | 125 | – | – | Verbrenn. |
| E | 250 | – | – | 15 |
| | 125 | – | – | 25 |
| unbehahdelte infizierte Pflanzen | | 100 | 100 | 100 |

die in den gleichen Konzentrationen angewendet wurden. Nach einer Inkubationszeit von 10 bis 14 Tagen wurden die Pflanzen auf Pilzbefall untersucht. Der Befallsgrad wurde ausgedrückt in % befallener Blattflächen, bezogen auf unbehandelte, infizierte Kontrollpflanzen (= 100 %).

Beispiel IV (bakterizide Wirksamkeit)

Jeweils 0,02 ml einer Bakteriensuspension von Xanthomonas malvacearum und Corynebacterium michiganense, wurden in Petrischalen auf Bakterien-Nähragar tropfenweise im Zentrum ausgebracht; dem Agar waren zuvor in noch flüssigem Zustand die in der Tabelle 5 beanspruchten Verbindungen in Wirkstoffkonzentrationen zugesetzt worden. Die beimpften Platten wurden nach 4 Tagen ausgewertet; hierbei wurde die Hemmung des Wachstums in % im Vergleich zur Kontrolle (= beimpfter Agar ohne Wirkstoffzusatz = 0 % Hemmung) ermittelt.

TABELLE 5

Bakterizide Wirksamkeit

| Verbindung (Beispiel) | Konzentration (ppm) | Hemmung in % von Xanthomonas malvacearum (gramnegativ) | Hemmung in % von Corynebacterium michiganense (grampositiv) |
|---|---|---|---|
| 11 a | 250 | 97 | 95 |
| (Na-Salz) | 125 | 70 | 50 |
| 12 a | 250 | 97 | – |
| | 125 | 80 | – |
| 1 a | 250 | – | 100 |
| | 125 | – | 100 |
| 11 a | 250 | – | 100 |
| (Säure) | 125 | – | 100 |

**Patentansprüche**

1. Imidazol-carbonsäuren und ihre Derivate der Formeln

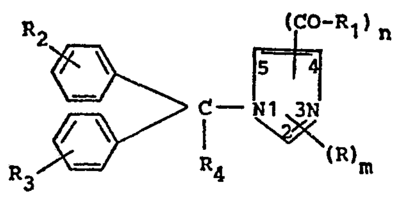

(I)

bzw.

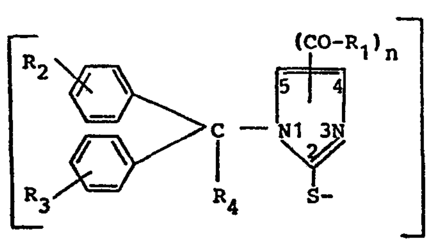

(II)

worin

m = 0 – 2; n = 1 oder 2 und m + n ≦ 3;

R Halogen, $(C_1-C_6)$Alkyl, Allyl, Hydroxy$(C_1-C_6)$alkyl, Halogen$(C_1-C_6)$alkyl, Mercapto, $(C_1-C_6)$Alkylthio, Cyano, Phenyl oder Phenyl$(C_1-C_2)$alkyl;

$R_1$ Hydroxy, $(C_1-C_6)$Alkoxy, Hydroxy$(C_2-C_6)$alkoxy, $(C_2-C_6)$-Alkoxy-alkoxy, Di$(C_1-C_3)$alkylphosphinyl-$(C_1-C_3)$alkoxy, Di$(C_1-C_3)$alkylphosphinyl-$(C_2-C_3)$hydroxyalkoxy, $(C_1-C_3)$Alkylthio, Amino, $(C_1-C_6)$-Alkylamino, Di$(C_1-C_6)$alkylamino, Di$(C_1-C_6)$alkylamino-$(C_1-C_3)$alkylamino, Hydroxyamino, $(C_1-C_3)$-Alkoxyamino; N-$(C_1-C_3)$-Alkyl-N-$(C_1-C_3)$-alkoxyamino, Anilino, N-Pyrrolidino, N-Piperidino, N-Morpholino, Hydrazino, N'-$(C_1-C_3)$-Alkylhydrazino, N',N'-Dimethylhydrazino oder N'-Phenylhydrazino,

$R_2$ und $R_3$ gleich oder verschieden sind und Wasserstoff, Halogen, $(C_1-C_3)$Alkyl, Trifluormethyl, Hydroxy, $(C_1-C_3)$Alkoxy, Halogen$(C_1-C_3)$alkoxy, $(C_1-C_3)$Alkylthio, Cyano, Nitro oder Acetamino, und

$R_4$ Wasserstoff oder Phenyl bedeuten sowie ihre nicht-toxischen Salze mit Säuren oder Basen.

2. Verbindung der Formel

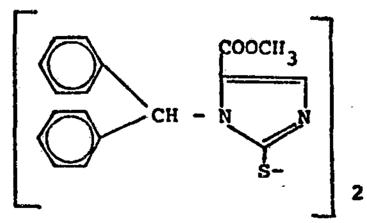

3. Verbindung der Formel

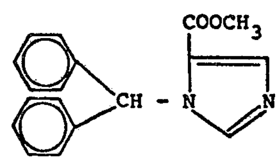

4. Verbindung der Formel

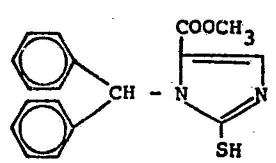

5. Verbindung der Formel

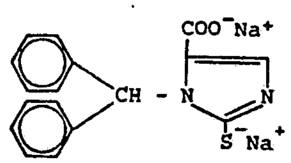

6. Verbindung der Formel

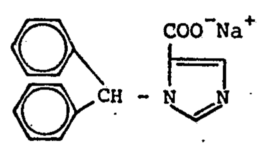

7. Verbindung der Formel

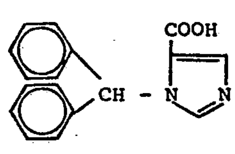

8. Verbindung der Formel

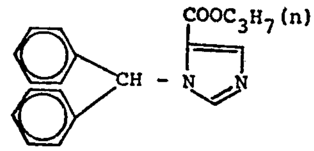

9. Verfahren zur Herstellung von Verbindungen der Formeln I bzw. II gemäß Anspruch 1, dadurch gekennzeichnet, daß man

a) Verbindungen der Formel

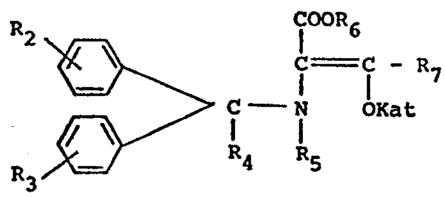

(III)

worin
$R_5$ Formyl oder Acetyl
$R_6$ $(C_1-C_6)$Alkyl
$R_7$ Wasserstoff oder $(C_1-C_6)$Alkoxycarbonyl und Kat ein Alkalimetallkation bedeuten, mit Rhodanwasserstoffsäure umsetzt und gewünschtenfalls in den erhaltenen 2-Mercapto imidazolen der Formel

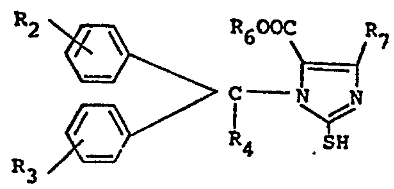

(IV)

die SH-Gruppe in 2-Stellung des Imidazolrings
$a_1$) unter Ausbildung einer -S-S-(Disulfid)-Brücke dehydriert oder
$a_2$) durch Entschwefelung eliminiert und anschließend gewünschtenfalls halogeniert oder in 2-Stellung hydroxy methyliert oder

b) Verbindungen der Formel

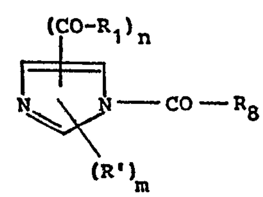

(V)

worin $R_8$ Wasserstoff oder $(C_1-C_3)$Alkyl bedeutet mit Verbindungen der Formel und R' die Bedeutung von R mit Ausnahme der -SH-Gruppe besitzt

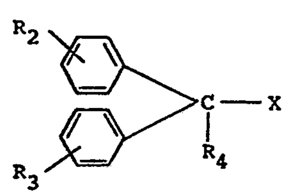

(VI)

worin X ein Halogenatom oder eine Alkyl- oder Arylsulfonylgruppe darstellt, umsetzt, anschließend die Gruppe $-CO-R_8$ in bekannter Weise abspaltet, oder

c) Verbindungen der Formel

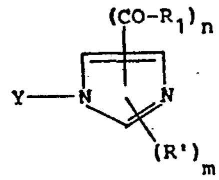

(VII)

worin Y Wasserstoff, ein Alkalimetall- oder Silberatom bedeutet, mit Verbindungen der Formel VI umsetzt, oder

d) Verbindungen der Formel

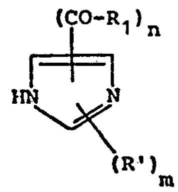

(VIII)

mit Verbindungen der Formel

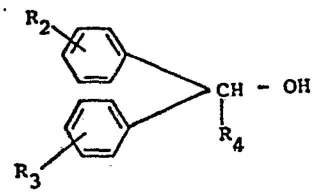

(IX)

umsetzt, oder

e) Verbindungen der Formeln

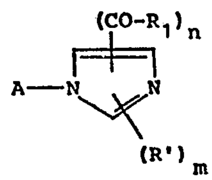

(X)

worin A eine Trimethylsilyl- oder Halogenmagnesiumgruppe bedeutet, mit Verbindungen der Formel

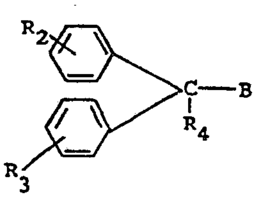

(XI)

worin B ein Halogenatom oder die Hydroxylgruppe darstellt, umsetzt, oder

f) Verbindungen der Formel

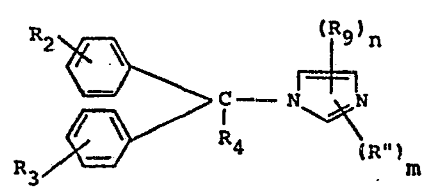

(XII)

worin R'' die Bedeutung von R mit Ausnahme der -SH- und der $(C_1-C_6)$Alkylthiogruppe besitzt und $R_9$ die Gruppen $-CH_2OH$ oder -CHO bedeutet, oxydiert, oder

g) in Verbindungen der Formel

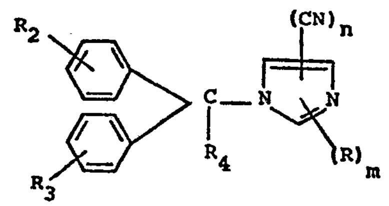

(XIII)

die -CN-Gruppe(n) zu(r) Carboxylgruppe(n) oder zu(r) Säureamidgruppe(n) hydrolysiert, und gewünschtenfalls in den nach a)—g) erhaltenen Verbindungen bis Estergruppe umestert oder verseift und/oder die erhaltenen freien Säuren gewünschtenfalls in nicht phytotoxische Salze, Ester, Thiolester, Amide, Anilide oder Hydrazide der Formeln I bzw. II überführt bzw. (bei Vorhandensein von mehreren Estergruppen) eine davon thermisch abspaltet.

10. Mittel, gekennzeichnet durch einen Gehalt an Verbindungen der Formeln I bzw. II gemäß Anspruch 1.

11. Verwendung von Verbindungen der Formeln I bzw. II gemäß Anspruch 1 zur Pflanzenwachstumsregulierung bzw. zum Pflanzenschutz.

## Revendications

1. Acides imidazole-carboxyliques et leurs dérivés qui répondent à l'une des formules

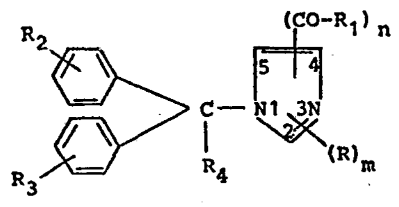

(I)

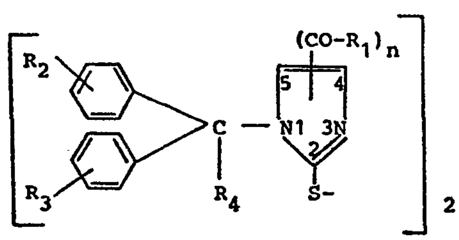

(II)

dans lesquelles:

m est un nombre de 0 à 2; n est égal à 1 ou à 2 et la somme (m + n) est au plus égale à 3;

R représente un halogène, un alkyle en $C_1-C_6$, un allyle, un hydroxy-alkyle en $C_1-C_6$, un halogéno-alkyle en $C_1-C_6$, un mercapto, un alkylthio en $C_1-C_6$, un cyano, un phényle ou un phényl-$(C_1-C_2)$alkyle;

$R_1$ représente un hydroxy, un alcoxy en $C_1-C_6$, un hydroxy-alcoxy en $C_2-C_6$, un $(C_2-C_6)$ alcoxy-alcoxy, un di-$(C_1-C_3)$alkylphosphinyl-$(C_1-C_3)$alcoxy, un di-$(C_1-C_3)$alkylphosphinyl-$(C_2-C_3)$hydroxy-alcoxy, un alkylthio en $C_1-C_3$, un amino, un $(C_1-C_6)$ alkylamino, un di-$(C_1-C_6)$alkylamino, un di-$(C_1-C_3)$alkylamino-$(C_1-C_3)$alkylamino, un hydroxy-amino, un alcoxy-amino en $C_1-C_3$, un N-$(C_1-C_3$-alkyl-N-$(C_1-C_3)$alcoxyamino, un anilino, un N-pyrrolidino, un N-pipéridino, un N-morpholino, un hydrazino, un N'-$(C_1-C_3)$alkyl-hydrazino, un N',N'-diméthyl-hydrazino ou un N'-phenyl-hydrazino,

$R_2$ et $R_3$ sont identiques ou différents et représentent chacun un hydrogène, un halogène, un alkyle un $C_1-C_3$, un trifluorométhyle, un hydroxy, un alcoxy en $C_1-C_3$, un halogéno-alcoxy en $C_1-C_3$, un alkylthio en $C_1-C_3$, un cyano, un nitro ou un acétylamino et

$R_4$ représente un hydrogéne ou un phényle, ainsi que les sels non toxiques qu'ils forment avec des acides ou des bases.

2. Composé de formule

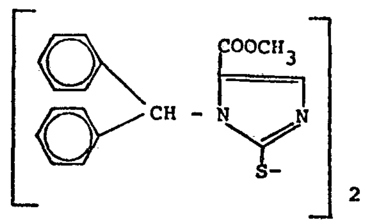

3. Composé de formule

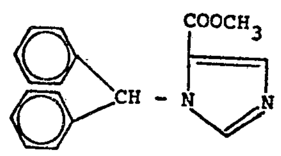

4. Composé de formule

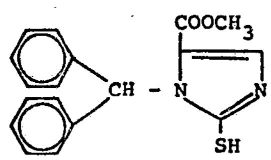

5. Composé de formule

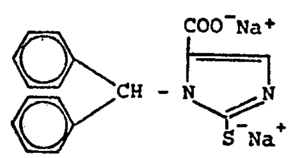

6. Composé de formule

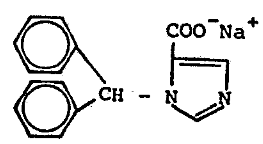

7. Composé de formule

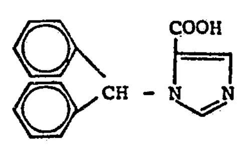

8. Composé de formule

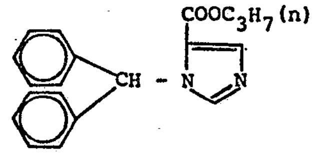

9. Procédé de préparation de composés de formules I ou II suivant la revendication 1, procédé caractérisé en ce que:

a) on fait réagir des composés répondant à la formule:

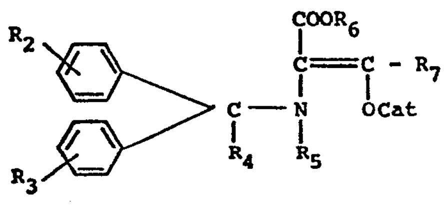

(III)

dans laquelle:
$R_5$ représente un radical formyle ou acétyle;
$R_6$ représente un radical alkyle un $C_1$—$C_6$,
$R_7$ représente un atome d'hydrogène ou un radical ($C_1$—$C_6$)alcoxy-carbonyle et Cat représente un cation de métal alcalin, avec l'acide thiocyanique et, si on le désire, dans les mercapto-2 imidazoles obtenus qui répondent à la formule:

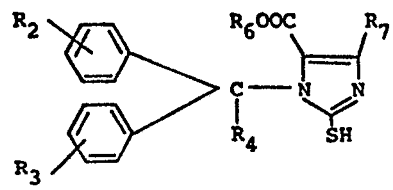

(IV)

a$_1$) On déshydrogène le groupe -SH qui se trouve en position 2 sur le noyau d'imidazole en formant un pont -S-S- (disulfure) ou

a$_2$) On élimine ce groupe -SH par désulfuration et ensuite, si on le désire, on halogène ou on hydroxyméthyle en position 2, ou

b) On fait réagir des composés qui répondent à la formule

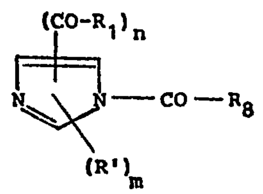

(V)

dans laquelle:
$R_8$ représente l'hydrogène ou un radical alkyle en $C_1$—$C_3$, et
R' a la signification de R à l'exception de groupe -SH,
avec des composés répondant à la formule:

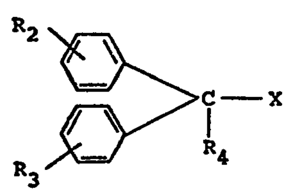

(VI)

dans laquelle:

X représente un atome d'halogène ou un radical alkyl- ou aryl-sulfonyle, puis on coupe le groupe -CO-$R_8$ de manière connue, ou

c) On fait réagir des composés répondant à la formule:

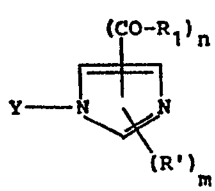

(VII)

dans laquelle:

Y représente l'hydrogène, un atome de métal alcalin ou un atome d'argent, avec des composés de formule VI, ou

d) On fait réagir des composés de formule:

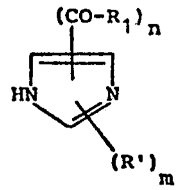

(VIII)

avec des composés de formule:

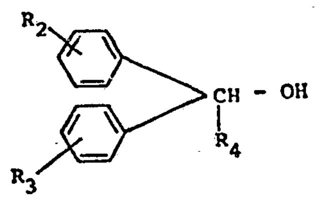

(IX)

ou

e) On fait réagir des composés qui répondent à la formule

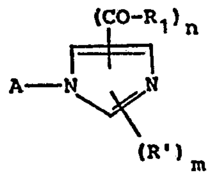

(X)

dans laquelle:

A représente un radical triméthylsilyle ou halogéno-magnésium, avec des composés répondant à la formule:

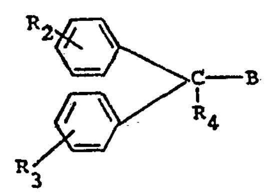

(XI)

dans laquelle:

B représente un atome d'halogène ou un group hydroxy,

ou

f) On oxyde des composés répondant à la formule:

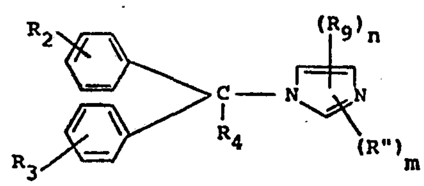

(XII)

dans laquelle:

R'' a la signification de R à l'exception du groupe -SH et du radical alkylthio en $C_1$—$C_6$ et $R_9$ représente un group $-CH_2OH$ ou -CHO,

ou

g) Dans des composés de formule:

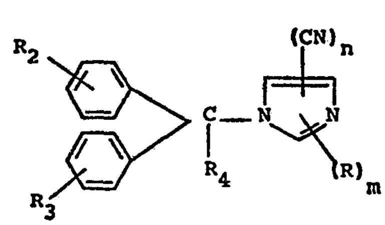

(XIII)

on hydrolyse le ou les groupes -CN en groupe(s) carboxy ou en group(s) amide(s), et, dans les composés obtenus selon a) à g), si on le désire, on transestérifie ou saponifie le groupe ester et/ou on transforme éventuellement les acides libres obtenus en sels, esters, esters de thiols, amides, anilides ou hydrazides de formule I ou II, non pyoto-toxiques, ou (lorsqu'il y a plusieurs groupes esters) on en coupe un par voie thermique.

10. Produits caractérisés en ce qu'ils contiennent des composés de formule I ou II selon la revendication 1.

11. Utilisation de composés de formule I ou II selon la revendication 1 pour régler la croissance de plantes ou pour protéger des plantes.

**Claims**

1. Imidazole-carboxylic acids and their derivatives of formulae

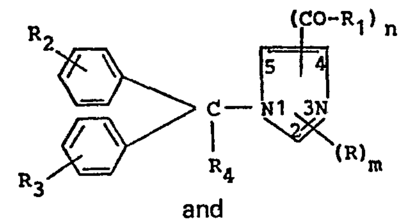

(I)

and

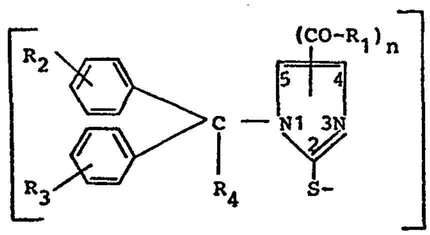

(II)

in which
m is zero, 1 or 2,
n is 1 or 2, and
m + n is equal to or smaller than 3;
R is halogen, $(C_1—C_6)$alkyl, allyl, hydroxy$(C_1—C_6)$alkyl, halo$(C_1—C_6)$alkyl, mercapto, $(C_1—C_6)$alkylthio, cyano, phenyl or phenyl$(C_1—C_2)$alkyl;
$R_1$ is hydroxy, $(C_1—C_6)$alkoxy, hydroxy$(C_2—C_6)$alkoxy, $(C_2—C_6)$alkoxyalkoxy, di$(C_1—C_3)$alkylphosphinyl$(C_1—C_3)$-alkoxy, di$(C_1—C_3)$alkylphosphinyl-$(C_2—C_3)$hydroxyalkoxy, $(C_1—C_3)$alkylthio, amino, $(C_1—C_6)$alkylamino, di$(C_1—C_6)$alkylamino, di$(C_1—C_3)$alkylamino-$(C_1—C_3)$alkylamino, hydroxyamino, $(C_1—C_3)$alkoxyamino; N-$(C_1—C_3)$-alkyl-N-$(C_1—C_3)$-alkoxyamino, anilino, N-pyrrolidino, N-piperidino, N-morpholino, hydrazino, N'-$(C_1—C_3)$alkylhydrazino, N',N'-dimethylhydrazino or N'-phenylhydrazino
$R_2$ and $R_3$, which can be identical or different, are hydrogen, halogen, $(C_1—C_3)$alkyl, trifluoromethyl, hydroxy, $(C_1—C_3)$alkoxy, halo-$(C_1—C_3)$-alkoxy, $(C_1—C_3)$alkylthio, cyano, nitro or acetamino, and
$R_4$ is hydrogen or phenyl, and the non-toxic salts thereof with acids or bases.

2. A compound as claimed in claim 1 having the formula

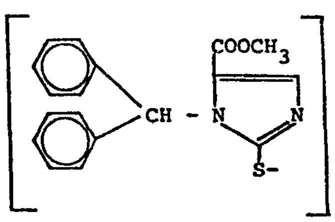

3. A compound as claimed in claim 1 having the formula

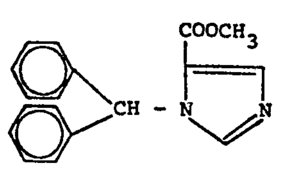

4. A compound as claimed in claim 1 having the formula

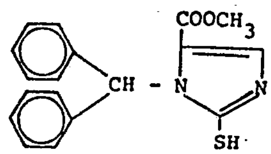

5. A compound as claimed in claim 1 having the formula

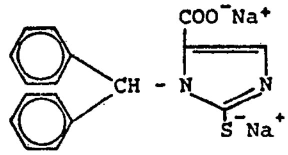

6. A compound as claimed in claim 1 having the formula

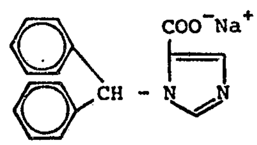

7. A compound as claimed in claim 1 having the formula

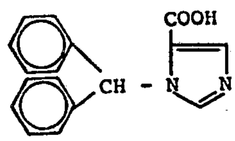

8. A compound as claimed in claim 1 having the formula

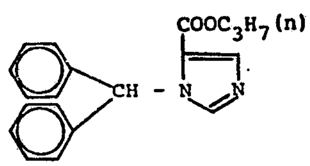

9. A process for the manufacture of compounds as claimed in claim 1, which comprises
a) reacting a compound of the formula

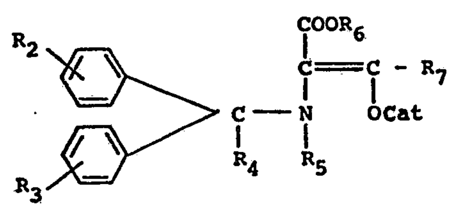

(III)

in which
$R_5$ is formyl or acetyl,
$R_6$ is $(C_1-C_6)$alkyl,
$R_7$ is hydrogen or $(C_1-C_6)$alkoxycarbonyl and Cat is an alkali metal cation with thiocyanic acid and, if desired, in the 2-mercapto-imidazoles of the formula

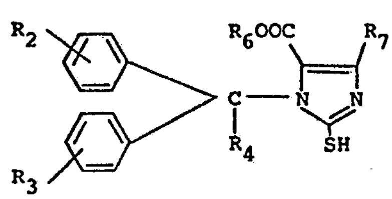

(IV)

thus obtained

$a_1$) dehydrating the SH group in 2-position of the imidazole ring with formation of a -S-S- (disulfide) bridge or

$a_2$) eliminating the SH group in 2-position of the imidazole ring by desulfurization, and if desired subjecting the products to halogenation or hydroxymethylation in 2-position, or

b) reacting a compound of the formula

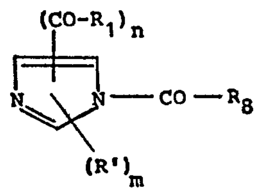

(V)

in which $R_8$ is hydrogen or ($C_1$—$C_3$)alkyl and R′ has the meaning of R except that it does not represent the -SH group, with a compound of the formula

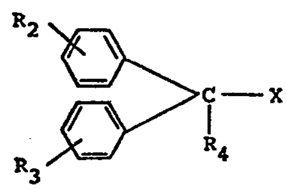

(VI)

in which X is a halogen atom or an alkyl or arylsulfonyl group and then splitting off the group -CO-$R_8$ in known manner, or

c) reacting a compound of the formula

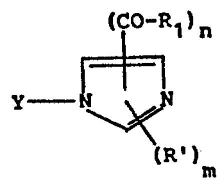

(VIII)

in which Y is hydrogen, an alkali metal or a silver atom with a compound of the formula VI, or

d) reacting a compound of the formula

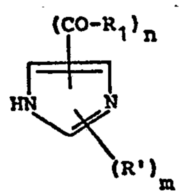

(VIII)

with a compound of the formula

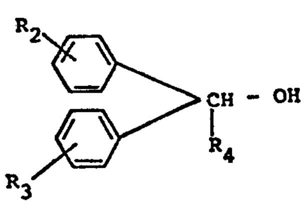

'(IX)

or

e) reacting a compound of the formula

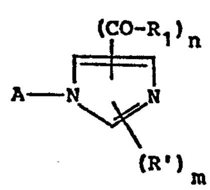

(X)

in which A is a trimethylsilyl or halomagnesium group with a compound of the formula

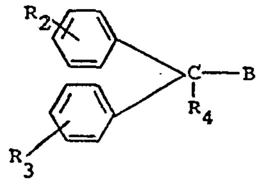

(XI)

in which B is a halogen atom or the hydroxy group, or

f) oxidizing a compound of the formula

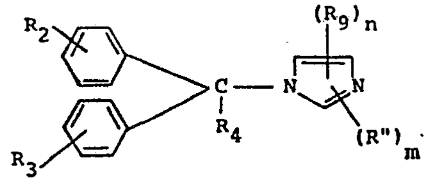

(XII)

in which R″ has the same meaning as R except that it cannot be -SH- or ($C_1$–$C_6$)alkylthio, and $R_9$ is $-CH_2OH$ or -CHO, or

g) in a compound of the formula

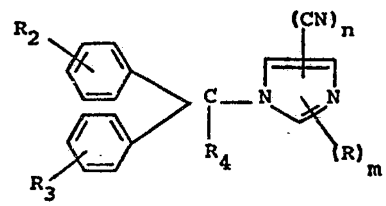

(XIII)

hydrolizing the -CN-groups(s) to form carboxyl group(s) or acid amide group(s) and, if desired, in the compounds obtained by reactions a) to g) transesterifying or saponifying the ester groups and/or transforming the free acids obtained into non-phytotoxic salts, esters, thioesters, amides, anilides, or hydrazides or formulae I or II, and, in the case of several ester groups being present, thermally splitting off one of them.

10. Composition containing an effective amount of a compound as claimed in claim 1.

11. Use of compounds as claimed in claim 1 as plant growth regulators or for plant protection.